# EUROPEAN PATENT APPLICATION

(11) **EP 3 382 013 A1**
(43) Date of publication of application: **03.10.2018**
(21) Application number: 17382151.3
(22) Date of filing: 27.03.2017
(51) Int. Cl.: C12N 9/08

(54) **MODIFIED UNSPECIFIC PEROXYGENASE AND USES THEREOF**

(71) Applicant: Repsol, S.A., 28045 Madrid (ES)
(72) Inventor: Rodríguez Buey, Marisa, 28935 Móstoles (ES); García Ruiz, Mónica, 28935 Móstoles (ES); Martín Díaz, Javier, 28049 Madrid (ES); Santos Moriano, Paloma Carmen, 28049 Madrid (ES); Molina Espeja, Patricia, 28049 Madrid (ES); García Ruiz, Eva, 28049 Madrid (ES); Plou Gasca, Francisco José, 28049 Madrid (ES); Alcalde Galeote, Miguel, 28049 Madrid (ES)
(74) Representative: ZBM Patents - Zea, Barlocci & Markvardsen

(57) **Abstract**

The present invention relates to a modified unspecific peroxygenase enzyme (UPO) EC 1.11.2.1 comprising: (a) a sequence having at least an 80% of sequence identity with the sequence SEQ ID NO: 2; or (b) a fragment of a sequence as defined in (a), said fragment maintaining the peroxygenase enzymatic activity; and further comprising at least one mutation in the turn-type motif defined by the amino acids 104 to 130 of the sequence SEQ ID NO: 2 and at least one mutation in the α-helix-type motif defined by the amino acids 186 to 196 of the sequence SEQ ID NO: 2.

The enzyme of the invention, which is thermostable, shows a significant increase in the activity towards oxidation reactions of two electrons and towards substrates such as styrene.

The present invention also relates to nucleic acids that encode the enzyme of the invention, as well as constructions, host cells and culture mediums containing them and the use thereof as a catalyst.

## Description

### Field of the invention

The present invention relates to peptides with unspecific peroxygenase activity, a nucleic acid encoding thereof, as well as nucleic acid constructions, vectors and host cells containing the nucleic acid, and to production methods and uses of the polypeptides.

### Background

The regioselective oxyfunctionalization of organic molecules is critical for synthesizing specific chemical products and raw materials, including functionalized polymers, pharmaceutical compounds and biofuels.

In most cases, this oxyfunctionalization is carried out under aggressive conditions that are not environmentally friendly. For example, the use of high temperatures and pressures (higher than 200°C and 30 atmospheres) in the cumene process for producing phenol or for transforming 1-naphthol from naphthalene is unfavorable in terms of energy use, releasing metal catalysts and toxic secondary products.

As an alternative to chemical catalysis for introducing oxygen functionalities, the possibility of using highly selective P450 monooxygenase-type enzymes which allowed to work at room temperature and atmospheric pressure in water and led to few secondary products, was proposed. Over the last couple of decades, these biocatalysts have been studied and modified to meet industrial standards; however, its nature as integral membrane protein , along with poor stability and the need for redox cofactors (NAD(P)H) and high-cost auxiliary flavoproteins, have prevented their practical application in many technological sectors.

Thirteen years ago the first unspecific peroxygenase (UPO, EC 1.11.2.1) was reported (Ullrich R. et al., 2004). This heme-thiolate enzyme is very stable and soluble, working as a self-sufficient mono(per)oxygenase fed by catalytic concentrations of H₂O₂. In fact, UPO can act by transferring an atom of oxygen from H₂O₂ into a large variety of compounds (more than 300 substrates tested to date), leading to the hydroxylation of aliphatic compounds, heterocyclic and aromatic oxygenations, as well as the epoxidation of alkenes, dealkylation, etc. (Hofrichter M. et al., 2014). Therefore, they have been studied to prepare active pharmaceutical ingredients (for example, in paracetamol and 4-hydroxy diclophenac synthesis), agrochemical products (including a number of pesticide precursors), and in food and cosmetic sectors as well as in bioremediation of xenobiotics

(including polycyclic aromatic hydrocarbons, PAHs).

*Agrocybe aegerita* UPO variants, showing improvements in terms of stability, production in heterologous expression systems or activity (cfr. Molina-Espeja P. et al., 2014), have been reported. These variants are based on modified regions with structural motifs of the same type, in particular, α-helix type.

Despite the interest shown in UPO, its low reaction specificity makes mandatory purification steps in order to eliminate the secondary products generated during the reaction, which means an increase in cost and time.

Therefore, despite the efforts made up to date, there is still the need of enzymes which are highly stable and show improved activity in the oxidation of a substrate of interest.

### Description of the invention

The inventors have surprisingly found that modulation of the enzyme activity towards oxidation of a substrate, in particular towards the epoxidation of styrene, is achieved by modifying the sequence of the unspecific peroxygenase enzyme EC 1.11.2.1 (hereinafter also referred to as "UPO") of *A. aegerita* in at least two motifs with different secondary structure.

According to the information obtained from the crystallographic structure of UPO1 enzyme of *A. aegerita* (PDB code: 2YP1) of sequence SEQ ID NO: 1, the secondary structure motifs are the following:

**Table 1**

| Motif | Position in SEQ ID NO: 1 | Motif | Position in SEQ ID NO: 1 | Motif | Position in SEQ ID NO: 1 |
|---|---|---|---|---|---|
| Turn | 48-78 | α-helix | 174-178 | Turn | 270-285 |
| α-helix | 79-87 | Turn | 179-183 | α-helix | 286-294 |
| Turn | 88-95 | α-helix | 184-199 | Turn | 295-298 |
| β sheet | 96-97 | Turn | 200-203 | β sheet | 299-300 |
| α-helix | 98-111 | α-helix | 204-223 | Turn | 301-308 |
| Turn | 112 | Turn | 224-226 | β sheet | 309-310 |
| α-helix | 113-129 | β sheet | 227-228 | Turn | 311-318 |
| Turn | 130-135 | α-helix | 229-239 | α-helix | 319-336 |
| β sheet | 136-137 | Turn | 240 | Turn | 337-339 |
| Turn | 138-141 | α-helix | 241-247 | α-helix | 340-357 |
| α-helix | 142-146 | Turn | 248-257 | Turn | 358-369 |
| Turn | 147-173 | α-helix | 258-269 | | |

The positions are provided with respect to SEQ ID NO: 1, which corresponds to the UPO sequence including the signal peptide

Until inventors' discovery, modifications in regions with the same secondary structure (in particular of α-helix type), with the aim of improving the enzyme profile in terms of stability, production in heterologous expression systems or activity, were reported in the state of the art (cfr. Molina-Espeja P. et al., 2014, supra).

The present inventors have found, however, a significant improvement in the epoxidation activity of the enzyme is achieved when two regions of the enzyme with different secondary structure are modified. In fact, the modified UPO enzyme of the invention shows increased activity towards styrene, the substrate towards which the natural form of the enzyme hardly shows affinity, and it efficiently oxidizes it. As shown in Table 7, mutating the sequence of an α-helix and a turn, an increase of 250% in the oxidation activity of styrene is achieved with respect to the enzymatic version that lacks these two mutations.

With this increase in the UPO enzyme activity, an increase in the yield of the epoxidation reaction is achieved, which simplifies and lowers the cost of producing derived products of interest (such as polyurethane) since (a) the production of secondary products associated with other enzymatic reactions is reduced, and (b) it is avoided or reduced the number of purification steps of the product of interest.

In addition, the use of the modified UPO of the invention in oxidation processes makes it possible to work in thermostable conditions that can be easily applied on an industrial scale.

In light of the foregoing, the present invention means a great advance forward in the chemical synthesis of polymers, biofuels or drugs, among others, on an industrial scale, since highly efficient oxyfunctionalization of organic molecules is achieved by using an enzyme that is of interest because (a) it works in gentle reaction conditions, which reduces energy costs, (b) it minimizes the formation of toxic reaction by-products, and (c) it is stable and soluble.

Thus, in a first aspect, the present invention provides a modified unspecific peroxygenase enzyme (UPO) EC 1.11.2.1 comprising:
(a) a sequence having at least an 80% of sequence identity with the sequence SEQ ID NO: 2; or
(b) a fragment of a sequence as defined in (a), said fragment maintaining the peroxygenase enzymatic activity;
and which further comprises at least one mutation in the turn-type motif defined by the amino acids 104 to 130 and at least one mutation in the α-helix-type motif defined by the amino acids 186 to 196 of the sequence SEQ ID NO: 2.

In a second aspect, the present invention provides a nucleic acid encoding an unspecific peroxygenase enzyme as defined in the first aspect of the invention.

In a third aspect, the present invention provides an expression cassette comprising the nucleic acid according to the second aspect of the invention, operatively linked to an expression promotor.

In a fourth aspect, the present invention provides an expression vector comprising the nucleic acid according to the second aspect of the invention, or the expression cassette according to the third aspect of the invention, operatively linked to an expression promotor.

In a fifth aspect, the present invention provides a host cell containing the construction according to the fourth aspect of the invention.

In a sixth aspect, the present invention provides a culture medium comprising the host cell as defined in the fifth aspect of the invention.

In a seventh aspect, the invention provides a kit comprising the unspecific peroxygenase enzyme as defined in the first aspect of the invention, or the nucleic acid as defined in the second aspect of the invention, or the expression cassette as defined in the third aspect of the invention, or the construction as defined in the fourth aspect of the invention.

In an eighth aspect, the present invention provides the use of an enzyme as defined in the first aspect of the invention as a catalyst.

Finally, in a ninth aspect, the present invention provides a catalytic oxidation method of a substrate which comprises carrying out the reaction of said enzymatic substrate in the presence of H₂O₂ and the enzyme as defined in the first aspect of the invention.

### Detailed description of the invention

As indicated above, the present invention provides a modified unspecific peroxygenase enzyme (UPO) EC 1.11.2.1.

The designation "EC 1.11.2.1" is recognized by a person skilled in the art as the EC number ("Enzyme Commission number") which corresponds to a numeric classification scheme for enzymes based on the chemical reactions that they catalyze. In the present case, the designation "EC 1.11.2.1" names the unspecific peroxygenase enzyme that catalyzes the reaction:

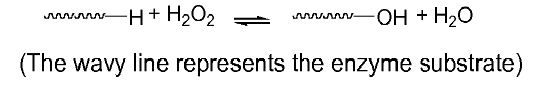

The enzymes belonging to this group are known for being heme-thiolate extracellular proteins of fungal origin. More than 1000 UPO sequences of different fungi such as *A. aegerita, Coprinellus radians*, *Coprinopsis cinerea*, *Marasmius rotula*, and *Sulfolobus tokodaii,* among others, have been identified and characterized. The sequences of these proteins are available in numerous genome databases. The enzymes of this group catalyze the insertion of an oxygen atom from H₂O₂ into a variety of substrates, including aromatic rings such as naphthalene, toluene, phenanthrene, pyrene and p-nitrophenol, recalcitrant heterocycles such as pyridine, dibenzofuran, various ethers (resulting in O-dealkylation) and alkanes such as propane, hexane and cyclohexane. The reactions that can catalyze the enzyme in a natural state include hydroxylation, epoxidation, N-oxidation, sulfooxidation, O- and N-dealkylation, bromination and one-electron oxidations.

In the present invention, the term "at least one mutation" is understood as at least one modification of the amino acid sequence in a specific motif of the protein, provided that the type of modification and number of modifications does not reduce or eliminate the oxidative enzymatic activity of the enzyme, and/or does not change its cell location or solubility when it is expressed.

The modification can consist of a substitution of one amino acid for another, the removal or insertion of an amino acid. In an embodiment of the first aspect of the invention, optionally in combination with any of the embodiments provided below, the modification consists of the substitution of one amino acid for another.

The amino acids can be classified by the group of the side chain. There are basically four different classes of amino acids based on the different nature of the side chains: (1) non-polar, (2) polar and neutral, (3) acidic and polar, (4) basic and polar.

The non-polar amino acids have side chains that are hydrocarbon alkyl groups (alkane branches) or aromatic (benzene rings) or heteroaromatic groups (for example, indole rings). Illustrative non-limitative examples of common non-polar amino acids are Ala, Val, Leu, Ile, Pro, Trp, Gly, Phe and Met.

Polar and neutral amino acids have polar but non-charged groups at neutral pH on the side chain (such as hydroxyl, amide or thiol groups). Illustrative non-limitative examples of polar and neutral amino acids are Ser, Thr, Cys, Tyr, Asn and Gin.

The acid amino acids (hereinafter also called "acidic and polar amino acids") have acidic side chains at neutral pH. They are aspartic acid or aspartate (Asp) and glutamic acid or glutamate (Glu), among others. Their side chains have carboxylic acid groups, the pKₐ of which is low enough to lose protons, being negatively charged in the process.

The basic amino acids (hereinafter also called "basic and polar amino acids") have side chains containing nitrogen and are similar to ammonia, which is a base (such as amines, guanidine or imidazole). Their pKₐ are high enough in such a way that they tend to bind protons, obtaining a positive charge in the process. Illustrative non-limitative examples of basic amino acids are Lys, Arg and His.

Any of the methods forming part of the knowledge of a person skilled in the art can be followed to modify the motif of the protein.

In an embodiment of the first aspect of the invention, optionally in combination with any of the embodiments provided below, the mutation consists of a conservative-type substitution, such as that amino acid of a motif having a specific secondary structure is substituted by another of the same nature (i.e. of the same group).

To carry out the conservative substitution in the amino acid sequence, any of the well-established protocols in the state of the art can be followed, such as, for example, directed mutagenesis, directed molecular evolution techniques including random mutagenesis, DNA shuffling, combinatorial saturation mutagenesis or neutral drift mutagenesis (Mate, D.M., et al., "The pocket manual of directed evolution: Tips and tricks. In: Biotechnology of Microbial Enzymes: Production, Biocatalysis and Industrial Applications", 2016, Brahmachari, Demain and Adrio Eds. Elsevier, pages 185-214).

The secondary structure of the proteins is the local folding between relatively close amino acid residues of the polypeptide chain. This type of protein structure is adopted thanks to the formation of hydrogen bonds between the carbonyl (-CO-) and amino (-NH-) groups of the carbon atoms involved in the close amino acid peptide bonds on the chain. They are also found in flat spiral shape. The main types of secondary structures are:
(a) Alpha helix: in this structure, the polypeptide chain is structured in a spiral shape over itself due to the turns produced around the beta carbon of each amino acid. This structure is maintained thanks to the intracatenary hydrogen bonds formed between the -C=O group of amino acid "n" and the -NH group of amino acid "n+4" (four amino acids down the chain). There are other types of helices: Helix 3₁₀ (hydrogen bonds between "n" and "n+3" amino acids) and helix n (hydrogen bonds between the "n" and "n+5" amino acids), however, they are much less common;
(b) Folded beta sheet (hereinafter also referred to as " β-sheet"): when the main chain is stretched to the maximum, covalent bonds can adopt a spatial configuration called beta sheet. Some protein regions adopt a zigzag structure and are connected to each another, establishing bonds via interstrand hydrogen bonds. All peptide bonds have these crossed bonds, thus giving the structure greater stability. The beta form is a simple shape formed by two or more parallel (running in the same direction) or non-parallel (running in opposite directions) peptide chains that are closely linked by means of hydrogen bonds and other types of steric interactions and constraints between the amino acid free radicals. This shape has a sheet folded structure, like an accordion; and
(c) Beta turns: Polypeptide chain sequences with an alpha or beta structure, often linked to each other by means of the so-called beta turns. They are short sequences, with a characteristic shape that creates a sharp 180-degree turn on the main polypeptide chain.

In another embodiment of the first aspect of the invention, optionally in combination with any of the embodiments provided above and below, the unspecific peroxygenase enzyme is from the fungal species *A. aegerita.*

Determining the peroxygenase activity can be carried out via any well-established protocol, such as those used in the experimental section below.

In the present invention, the term "identity" refers to the percentage of identical residue in two sequences when they are optimally aligned. If, in the optimal alignment, a position in the first sequence is occupied by the same amino acid residue as the corresponding position in the second sequence, the sequences show identity with respect to that position. The identity level between two sequences (or "percent sequence identity") is measured as a ratio of the number of identical positions shared by the sequences to the size of the sequences (that is, the percent sequence identity = (number of identical positions / total number of positions) x 100).

A large number of mathematical algorithms that make it possible to quickly obtain optimal alignment and calculate identity between two or more sequences are known, said algorithms being incorporated into a series of available software programs. Examples of these programs for analyzing amino acid sequences include, among others, the programs MATCH-BOX, MULTAIN, GCG, FASTA and ROBUST. Preferred analysis software programs include ALIGN, CLUSTAL W and the BLAST programs (for example, BLAST versions 2.1, BL2SEQ and subsequent ones thereof). To analyze amino acid sequences, a weight matrix is used, such as the BLOSUM matrix (for example, the BLOSUM45, BLOSUM50, BLOSUM62 and BLOSUM80 matrix), the Gonnet matrices, or the PAM matrices (for example, PAM30, PAM70, PAM120, PAM160, PAM250, and PAM350 matrices).

The BLAST programs analyze at least two amino acid sequences, either by aligning a sequence selected with multiple sequences in a database (for example, GenSeq) or with BL2SEQ, between two selected sequences. The BLAST programs are preferably modified by low-complexity filter programs, such as the DUST or SEG programs, which are preferably integrated into the functions of the BLAST program. If a penalty is applied to the score due to the lack of alignment that generates a gap therein (gap score), the value thereof is preferably between -5 and -15. Similar gap parameters can be used with other programs, as appropriate. The BLAST programs and the underlying principles thereof are described in greater detail in, for example, Altschul et al., "Basic local alignment search tool.", 1990, J.Mol. Biol, v. 215, pages 403-410.

To analyze the multiple sequences, the CLUSTAL W program can be used. The CLUSTAL W program is desirably carried out using the "dynamic" (instead of the "fast") mode. The amino acid sequences are evaluated using a variable set of BLOSUM matricies based on the identity level between the sequences. The CLUSTAL W program and the underlying principles are also described in, for example, Higgins et al. 1992.

In one embodiment, optionally in combination with any of the embodiments provided above and below, the sequences of the first aspect of the invention have a sequence identity of at least 80%, 81%, 82%, 83%, 84%, 85%, 86%, 87%, 88%, 89%, 90%, 91%, 92%, 93%, 94%, 95%, 96%, 97%, 98%, or 99% with the reference sequence (either SEQ ID NO: 2, referred to above, as well as ID NO: 3 and 4 referred to below).

In another embodiment of the first aspect of the invention, optionally in combination with any of the embodiments provided above and below, the unspecific peroxygenase enzyme has a mutation in position 127 referring to SEQ ID NO: 2.

In another embodiment of the first aspect of the invention, optionally in combination with any of the embodiments provided above and below, the enzyme has a mutation in position 191 referring to SEQ ID NO: 2.

In another embodiment of the first aspect of the invention, optionally in combination with any of the embodiments provided above and below, the unspecific peroxygenase enzyme is as sequence SEQ ID NO: 2 and has a mutation in the turn-type motif defined by the amino acids 104 to 130 and a mutation in position 191, the positions being with respect to the sequence SEQ ID NO: 2. In one embodiment of the first aspect of the invention, optionally in combination with any of the embodiments provided above and below, the unspecific peroxygenase enzyme is as sequence SEQ ID NO: 2 and has a mutation in position 127 and a mutation in the motif defined by the amino acids 186 to 196, the positions referring to the sequence SEQ ID NO: 2. In one embodiment of the first aspect of the invention, optionally in combination with any of the embodiments provided above and below, the unspecific peroxygenase enzyme is of sequence SEQ ID NO: 2 and has a mutation in position 127 and a mutation in position 191, the positions referring to the sequence SEQ ID NO: 2.

In another embodiment of the first aspect of the invention, optionally in combination with any of the embodiments provided above and below, the mutation in each of the domains consists of a substitution by another amino acid. In another embodiment of the first aspect of the invention, optionally in combination with any of the embodiments provided above and below, the mutation in each of the motifs consists of the substitution by a non-polar amino acid, with D or L configuration. In one embodiment of the first aspect of the invention, optionally in combination with any of the embodiments provided above and below, the substitution is by a non-polar L-amino acid. In another embodiment of the first aspect of the invention, optionally in combination with any of the embodiments provided above and below, the substitution is by an amino acid selected from Ala, Val, Leu and Ile. In another embodiment of the first aspect of the invention, the substitution is by Leu or Ile, either with D or L configuration.

In another embodiment of the first aspect of the invention, optionally in combination with any of the embodiments provided above and below, the unspecific peroxygenase enzyme has the sequence SEQ ID NO: 2 and has the mutation Met127Ile.

In another embodiment of the first aspect of the invention, optionally in combination with any of the embodiments provided above and below, the unspecific peroxygenase enzyme has the sequence SEQ ID NO: 2 and has the mutation Phe191 Leu.

In another embodiment of the first aspect of the invention, optionally in combination with any of the embodiments provided above and below, the unspecific peroxygenase enzyme is selected from the group consisting of:
(c) a sequence having at least an 80% of sequence identity with the sequence SEQ ID NO: 3 (which corresponds to the UPO sequence SEQ ID NO: 2 with the mutations Met127Ile and Phe191 Leu); and
(d) a fragment of the sequence (c) provided that the fragment shows the enzymatic activity and mutations in positions Met127Ile and Phe191 Leu of SEQ ID NO: 3.

In another embodiment of the first aspect of the invention, optionally in combination with any of the embodiments provided above and below, the enzyme has the sequence SEQ ID NO: 3.

In another embodiment of the first aspect of the invention, optionally in combination with any of the embodiments provided above and below, the unspecific peroxygenase enzyme further comprises the substitution of at least one Ser residue by one Cys residue. In another embodiment of the first aspect of the invention, optionally in combination with any of the embodiments provided above and below, the unspecific peroxygenase enzyme further comprises the substitution of a Ser residue by Cys residue. In another embodiment of the first aspect of the invention, optionally in combination with any of the embodiments provided above and below, the unspecific peroxygenase enzyme comprises the substitution of Ser residue, which is outside the catalytic region, by a Cys residue. In another embodiment of the first aspect of the invention, optionally in combination with any of the embodiments provided above and below, the unspecific peroxygenase enzyme comprises the substitution of Ser residue, which is on the opposite side of the catalytic region, by a Cys residue. In another embodiment of the first aspect of the invention, optionally in combination with any of the embodiments provided above and below, the unspecific peroxygenase enzyme has the sequence SEQ ID NO: 2 or 3 and the Ser residue is substituted in position 221 of SEQ ID NO: 2 or SEQ ID NO: 3 by a Cys residue. As mentioned above, the substitution of Ser by Cys can be carried out by routine methods. By way of illustration, in the examples provided below, the substitution of Ser by Cys through directed mutagenesis is illustrated.

In another embodiment of the first aspect of the invention, optionally in combination with any of the embodiments provided above and below, the unspecific peroxygenase enzyme has a sequence selected from the group consisting of:
(e) a sequence having at least an 80% of sequence identity with the sequence SEQ ID NO: 4 (which corresponds to the native UPO sequence SEQ ID NO: 2 with the mutations Met127Ile, Phe191Leu and Ser221Cys); and
(f) a fragment of the sequence (e) provided that the fragment shows the enzymatic activity and the mutations in the positions Met127Ile, Phe191Leu and Ser221Cys of SEQ ID NO: 4.

In another embodiment of the first aspect of the invention, optionally in combination with any of the embodiments provided above and below, the enzyme has the sequence SEQ ID NO: 4.

In another embodiment of the first aspect of the invention, optionally in combination with any of the embodiments provided above and below, the unspecific peroxygenase enzyme further comprises one or more mutations selected from the group consisting of Va157Ala, Leu67Phe, Val751Ile, Ile248Val, and Phe311 Leu, the positions referring to SEQ ID NO: 2, 3 or 4.

In another embodiment of the first aspect of the invention, optionally in combination with any of the embodiments provided above and below, the peroxygenase enzyme further comprises the mutations Val57Ala, Leu67Phe, Val75Ile, Ile248Val, and Phe311Leu, the positions referring to SEQ ID NO: 2, 3 or 4.

In one embodiment of the first aspect of the invention, optionally in combination with any of the embodiments provided above and below, the enzyme comprises a signal peptide sequence at its amino-terminal end.

In the present invention, the term "signal peptide" is understood as an amino acid sequence located at the amino end of a protein or peptide, whose function is to direct the location of the protein in a specific cellular compartment or intra- or extracellular space.

In another embodiment of the first aspect of the invention, optionally in combination with any of the embodiments provided above and below, the enzyme comprises the signal peptide of sequence SEQ ID NO: 5 at its amino-terminal end. In another embodiment of the first aspect of the invention, optionally in combination with any of the embodiments provided above and below, the enzyme comprises the sequence SEQ ID NO: 2, 3 or 4, and the signal peptide of the sequence SEQ ID NO: 5 at its amino-terminal end.

The inventors have found that when the enzyme of the first invention is immobilized in a solid support, the epoxidation activity is increased even more. From the results shown in Table 8, it is concluded that when the enzyme of the invention, including a mutation in an α-helix type structure domain and a second mutation in a turn-type structure domain, is immobilized, there is a 50% increase compared to the same enzymatic structure without immobilization, and a 100% increase compared to a version of an enzymatic structure that does not include the mutations in the structural motifs.

Thus, in one embodiment of the first aspect of the invention, the enzyme is immobilized. There are well-known protocols in the state of the art for immobilizing a protein. Illustrative and non-limitative examples of enzyme immobilization protocols are:
(a) Entrapment: physical retention of the enzyme in the inner cavities of a solid, porous matrix generally made up of photocrosslinkable prepolymers or polyacrylamide-type polymers, collagen, alginate, carrageenan or polyurethane resins. The immobilization process is carried out by means of suspending the enzyme in a monomer solution. Polymerization is then initiated. Entrapment can occur in gels or fibers, which are usually more resistant that the gels;
(b) Microencapsulation: the enzymes are surrounded by semipermeable membranes allowing substrate and product molecules to pass, but not those of the enzyme. These semipermeable membranes can be permanent (caused by interfacial polymerization) or non-permanent (created by surfactants, also called "reverse micelles"). The microcapsules obtained have a spherical shape, with diameter sizes between 1 and 100 µm;
(c) Membrane reactors: permeable membranes are applied to the final product, permeable or impermeable membranes to the initial substrate, and impermeable to the enzyme. A liquid substrate flow crossing the reactor is established via a pump. In general, this methodology first starts with the adsorption of the enzyme on the membrane that will form the reactor. This adsorption can be carried out in two ways: (i) by passing an enzyme buffer solution through the membrane; (ii) by continuous contact of the enzyme solution with the membrane; and
(d) by attachment to a solid support: there is a wide variety of support materials for enzyme immobilization. These materials have different sizes, densities, porosities and shapes, although they are generally found with the shape of a cylinder, sheets, fibers and, more commonly, in the shape of spheres. The supports can be classified in two large groups: (1) inorganic supports: natural (clays such as bentonite, pumice stone, silica, etc.) or manufactured materials (metal oxides and glass with a controlled pore size, non-porous glass, alumina, ceramics, silica gel, etc.); and (2) organic supports: natural polymers, subdivided into: polysaccharides (cellulose, starch, dextrans, agar, agarose, alginates, chitin, chitosan, etc.) and fibrous proteins (collagen, keratin, etc.), among others; and synthetic polymers, divided into: polyolefins (such as polystyrene), acrylic polymers (polyacrylates, polyacrylamides, polymethacrylates, etc.) and others, such as polyvinyl alcohol, polyamides, etc. The enzyme can be attached to the support by means of adsorption or covalent bond.

A person skilled in the art, using general knowledge and routine protocols, can immobilize the enzyme of the invention choosing the type of support and specific conditions.

In an embodiment of the first aspect of the invention, optionally in combination with any of the embodiments provided above and below, the enzyme is immobilized in a solid support. In another embodiment of the first aspect of the invention, optionally in combination with any of the embodiments provided above and below, the enzyme is immobilized in a solid support by means of a covalent bond. In another embodiment of the first aspect of the invention, optionally in combination with any of the embodiments provided above and below, the enzyme has a mutation of Ser by Cys and it is immobilized in a solid support by a -S-S-type covalent bond.

In a second aspect, the present invention provides a nucleic acid encoding for the enzyme defined in the first aspect of the invention and the particular embodiments of said first aspect.

The nucleic acid encoding the enzyme can include, optionally, a coding sequence of the signal peptide.

In another embodiment of the second aspect of the invention, optionally in combination with any of the embodiments provided above and below, the signal peptide corresponds to the signal peptide of the peroxygenase enzyme of the invention. In another embodiment of the second aspect of the invention, optionally in combination with any of the embodiments provided above and below, the nucleic acid comprises the sequence SEQ ID NO: 6 which encodes for the signal peptide of the amino acid sequence SEQ ID NO: 5.

In a third aspect, the present invention provides an expression cassette comprising the nucleic acid of the second aspect of the invention.

"Expression cassette" is a nucleic acid molecule, either single- or double-stranded, that contains the control sequences required for expressing a coding sequence of the present invention.

In the present invention, the term "control sequence" is defined to include all components that are necessary or advantageous for expressing the coding sequence of the nucleic acid sequence. Each control sequence can belong to or be external to the nucleic acid sequence encoding the polypeptide. Such control sequences include, but are not limited to, a leader sequence, a polyadenylation sequence, a propeptide sequence, a promoter, a signal sequence, and a transcription terminator. The control sequences include, at least, a promoter and transcriptional and translational stop signals. The control sequences can be provided with specific sequences that make it possible to generate specific restriction sites that make it easier the operatively linking of the control sequences with the coding region of the nucleic acid sequence encoding a polypeptide.

In a fourth aspect, the present invention provides an expression vector.

In the expression vector, the nucleic acid sequence encoding the enzyme of the invention should be operatively linked to suitable promoter and terminator sequences. The promoter can be any DNA sequence showing transcriptional and translational activity in the selected host cell and can be derived from genes encoding proteins that are homologous or heterologous to the host cell. The methods used to link the DNA sequences encoding the enzyme, the promoter and the terminator and insert them into the correct vectors are known by those skilled in the art.

The recombinant expression vector can be any vector (for example, a plasmid or virus) that can be conveniently subjected to recombinant DNA methods and can lead to the expression of the nucleic acid sequence.

More than one copy of a nucleic acid encoding the enzyme in the host cell can be inserted in order to amplify the expression of the nucleic acid sequence. Stable amplification of the nucleic acid sequence can be obtained by integrating at least one additional copy of the sequence in the host cell genome using well-known methods in the state of the art and selecting transformants that contain it.

The methods used to link the previously described elements to construct the recombinant expression vectors of the present invention are known by a person skilled in the art.

In a fifth aspect, the present invention provides a cell host comprising the expression cassette of the third aspect or the expression vector of the fourth aspect of the invention.

As used in the present specification, a "host cell" includes any cultivable cell that can be modified by means of introducing a nucleic acid that is not naturally contained in the cell. And wherein the polynucleotide of the invention can be expressed, leading to a stable polypeptide, modified post-translationally and located in the proper subcellular compartment. The selection of a suitable host cell can also be influenced by the selection of the detection signal. For example, the use of constructions with reporter genes (for example, lacZ, luciferase, thymidine, kinase or GPF) can provide a selectable signal by activating or inhibiting transcription of the gene of interest in response to a transcription regulatory protein.

A host cell of the present invention includes prokaryotic and eukaryotic cells. The prokaryotes include gram-negative (for example, *Escherichia coli*) or gram-positive organisms (for example, bacteria of the genus *Bacillus* sp.). The prokaryotic cells will be optionally used to propagate the transcription control sequence of the vector that contains the polynucleotide(s) object of the invention, which will make it possible to obtain a greater number of copies of the vector containing the polynucleotide(s) object of the invention. Among the prokaryotic host cells suitable for the transformation of this vector, there are, for example, *E. coli, Bacillus subtilis*, *Salmonella typhimurium*, and other species within the genera *Pseudomonas, Streptomyces* and *Staphylococcus.* The eukaryotic cells include, among others, yeast cells, plant cells, fungal cells, insect cells, mammal cells and parasitic organism cells (for example, *Trypanosomas*). As used in the present document, the term "yeast" does not only include yeast in the strictly taxonomic sense, i.e., unicellular organisms, but also multicellular fungi similar to yeasts or filamentous fungi. Examples of species are *Kluyveromyces lactis, Schizosaccharomyces pombe,* and *Ustilago maydis*, with *Saccharomyces cerevisiae* and *Pichia pastoris* as preferred organisms. Other yeasts that can be used for producing the polyamino acid sequence(s) of the present invention are *Neurospora crassa, Aspergillus niger*, *Aspergillus nidulans*, *Aspergillus sojae*, *Aspergillus oryzae*, *Candida tropicalis*, and *Hansenula polymorpha.* The cultivation systems with mammal host cells include cells fromcell lines established as COS cells, L cells, 3T3 cells, Chinese hamster ovary (CHO) cells, embryonic stem cells, being preferred BHK, HeK or HeLa cells. The eukaryotic cells are preferably used to express the recombinant gene by applying the regulatory sequence of transcription or the expression vector of the present invention.

The method for obtaining the enzyme of the invention from the transformed host cell comprises the steps: (a) Introducing the vector of the invention as previously described in a suitable host cell (host cell of the invention), b) cultivating the host cell of the invention in a suitable medium, and c) purifying the polypeptide of the invention with peroxygenase activity.

The terms "purify", "isolate", "isolation" or "purification" of the enzyme refers to the separation of the protein and, alternatively, the concentration thereof, from the culture medium of the cell of the invention. The methods for separating and purifying polypeptides are well-known in the state of the art, including, but not limited to, differential solubility techniques, electrophoresis or isoelectric focusing. For some purposes, it is preferable to produce the polypeptide in a recombinant system wherein the protein contains an additional sequence thought to facilitate purification, such as, but not limited to, a polyhistidine sequence. The chromatography techniques can be based on the molecular weight, charge or affinity of the protein and can be carried out through a column, on paper or on a plate. The protein separation can be performed, for example, by fast protein liquid chromatography (FPLC) in an automated system that notably reduces the purification time and increases the purification yield.

The expression "cultivating host cells" refers to the process of maintaining and growing the host cells.

The cell is preferably transformed with a vector comprising a nucleic acid sequence of the invention followed by the integration of the vector in the host chromosome.

In a sixth aspect, the present invention provides a culture medium comprising the host cell as defined in the fifth aspect of the invention.

Selecting a particular culture medium, as well as the culture conditions, which will depend on the host cell, form part of the routine work of the person skilled in the art.

In a seventh aspect, the invention provides a kit comprising the unspecific peroxygenase enzyme as defined in the first aspect of the invention, or the nucleic acid as defined in the third aspect of the invention, or the construct as defined in the fourth aspect of the invention.

In an embodiment of the seventh aspect of the present invention, the kit comprises the unspecific peroxygenase enzyme of the first aspect of the invention in freeze-dried form. The enzyme of the invention can be freeze-dried according to the techniques well-known in the state of the art.

In another embodiment of the seventh aspect of the present invention, the kit comprises the unspecific peroxygenase enzyme of the first aspect of the invention suspended in a buffer. There are many buffers on the market used in the suspension, preservation and storage of the enzyme of the invention, such as the enzyme storage buffer.

In an embodiment of the seventh aspect of the present invention, the kit further comprises instructions for the use thereof as a catalyst.

In an eighth aspect, the present invention provides the use of an enzyme as defined in the first aspect of the invention as a catalyst.

In an embodiment of the eighth aspect of the present invention, it is provided the use of the enzyme, as defined in the first aspect of the invention and in the embodiments provided above, as a catalyst of a substrate oxidation reaction.

In another embodiment of the eighth aspect of the present invention, it is provided the use of the enzyme as defined in the first aspect of the invention and in the embodiments provided above, as a catalyst of a styrene oxidation reaction.

Lastly, in a ninth aspect, the present invention provides a method for the catalytic oxidation of a substrate, which comprises carrying out the reaction of said enzymatic substrate in the presence of a catalytic concentration of H₂O₂ and the enzyme as defined in the first aspect of the invention or in any of the embodiments provided above.

In one embodiment of the ninth aspect of the invention, the substrate is styrene.

In the present invention, the expression "catalytically effective amount" refers to the amount of H₂O₂ that is less than the stoichiometric amount in the initial quantity and that is sufficient for the enzymatic reaction to take place.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### Analysis methods

### 1. Catalytic promiscuity measurement

As mentioned above, one of the contributions of *neutral drift* is the ability to obtain mutants that have improved activities that are very different from those evaluated during the screening tests. For this reason, the activity of enzymatic supernatants on 3 different substrates was measured: ABTS (2,2'-azino-bis(3-ethylbenzothiazoline-6-sulphonic acid) used during the screening) (Sigma-Aldrich), NBD (5-nitro-1,3-benzodioxole) (Tokyo Chemical Industry), and styrene (Sigma-Aldrich) (Diagram 1). These three substrates were selected since they involve a wide range of catalytic activities carried out by the UPO. This enzyme is able to carry out oxidation reactions of a single electron, e.g. ABTS E°=+0.69 V, as well as oxidation reactions of two electrons (oxygen insertion), such as dealkylation (e.g. of NBD) or epoxidation (styrene). In the particular case of styrene, the disappearance thereof and the formation of styrene oxide was measured by GC/MS, while colorimetric methods were used for the other tested substrates.

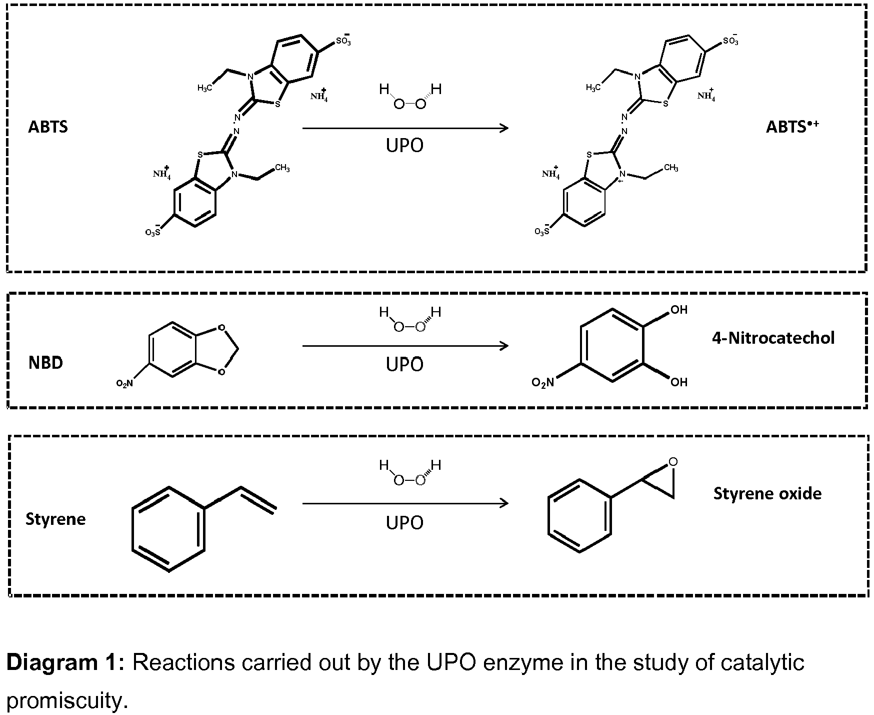

### 1.1. Activity test with ABTS in kinetic mode

20 µL of enzymatic supernatant was transferred to 96-well plates, wherein 180 µL of the reaction medium was introduced: 0.3 mM ABTS, 2 mM H₂O₂, 100 mM citrate-phosphate buffer at pH 4.4. The activity was measured by continuously measuring the increase in absorbance at 418 nm (kinetic mode) for 5 minutes (Spectra Max Plus 384, Molecular Devices, USA). The clone activities were made relative with regard to the parental type.

### 1.2. Activity test with NBD

The measurement for NBD was carried out with 20 µL of enzymatic supernatant and 180 µL of reaction medium: 1 mM NBD in acetonitrile (20%) and 1 mM H₂O₂ in 100 mM potassium phosphate buffer at pH 7.0. The activity was measured in the same way as the test for ABST, measuring the absorbance at 425 nm.

### 1.3. Activity test with styrene

200 µL of enzyme was added to a vial of 5 mL to which 800 µL of reaction medium was added: 1 mM styrene in methanol (20%), 100 mM sodium phosphate buffer at pH 7.0; 1 mM H₂O₂. The reaction was incubated at room temperature while stirring. After 1 hr of reaction, 500 µL of sample were extracted in a new vial, adding 500 µL of chloroform. After 20 minutes of stirring, the organic phase was extracted and 1µL of sample was injecting in splits in a (GC/MS) (Saturn 2000, Variant). The injection was carried out at 90°C following the temperature ranges below: 35°C (8 min), gradient of 35-80°C (5°C/min) and 80°C (2 min). The activity was measured by calculating the ratio between the disappearance of the styrene and the appearance of styrene oxide.

### 2. High-throughput screening (HTS) protocols

### 2.1. Screening

Individual colonies of *S*. *cerevisiae* BJ5465 (ATCC 208289, LGC Promochem), of each of the generations were selected and transferred to 96-well plates containing 50 µL with a minimum selective medium without uracil (SC). The SC medium is made up of 6.7 g/L of YNB (Yeast Nitrogen Base) medium without amino acids (Difco), 1.92 g/L of amino acid supplement without uracil (Sigma-Aldrich,), 20 g/L of raffinose (Sigma-Aldrich) and 25 mg/L of chloramphenicol (Sigma-Aldrich). The H1 well was inoculated with S. *cerevisiae* without transforming as a negative control, while column 6 was inoculated with the parental type (mutant PaDa-I of UPO1) in all the generations. The plates were sealed and incubated at 30°C, 250 rpm and 80% relative humidity (*Minitron-INFORS.* Biogen, Spain) for 48 h. Once this time has elapsed, 160 µL of expression medium was added to each one of the wells, incubating the clones for 48 hrs under the same conditions. The expression medium is made up of 10 g/L of yeast extract (Becton, Dickinson and Company); 20 g/L of Bacto Peptone (Becton, Dickinson and Company); 60 mM KH₂PO₄ buffer (Sigma-Aldrich) at pH 6.0; 20 g/L of galactose (Sigma-Aldrich); 2 mM MgSO₄ (Sigma-Aldrich); 25 g/L of ethanol (Mervilab) and 25 mg/L of chloramphenicol.

The measurement of the activity was carried out in HTS format: 20 µL of supernatant were transferred to new 96-well plates where the activity was measured by adding 180 µL of reaction medium as described in section 1.1.

### 2.2. First re-screening

A total of 33 clones of the last generation were selected, which were fermented in optimal conditions in order to carry out catalytic promiscuity trials against different substrates. 20 µL of culture of each clone were transferred to 5 mL of SC medium. The parental (PaDa-I mutant) and a negative control (non-transformed *S*. *cerevisiae*) were grown in the same way. After 48 hr, the OD₆₀₀ of the cultures was measured by carrying out a refresh in SC medium, leaving the final concentration at 0.3. After 6 hrs (OD₆₀₀=1), 1 mL of the culture was added to flasks of 100 mL containing 9 mL of supplemented expression medium (made up of 10 g/L of yeast extract; 20 g/L of Bacto Peptone; 60 mM KH₂PO₄ buffer at pH 6.0; 20 g/L of galactose; 2 mM MgSO₄; 25 g/L of ethanol, 300 mg/L hemoglobin and 25 mg/L of chloramphenicol). The flasks were grown at 30°C and 250 rpm for 48 hrs, time after which the cultures were centrifuged and the enzymatic supernatant collected.

The activity measurement was carried out in triplicate, with 20 µL of enzymatic supernatant, as described in section 1.1.

### 2.3. Second re-screening

1.5 mL of culture was collected from the inoculum in SC medium from the first re-*screening*, and an extraction of the plasmid using the *Zymoprep* kit (Zymo Research) was carried out. Once the genetic material is obtained, it was transformed into competent *E. coli* XL2-Blue cells (Stratagen) in Luria-Bertani plates with 10 mg/L of ampicillin (LB/ampicillin). The LB medium contains 10 g/L of peptone, 5 g/L of yeast extract and 5 g/L of NaCl (Panreac); pH 7.5 adjusted with NaOH 1 N, and the mixture is sterilized for 20 mins at 121°C and 1.5 bar. After sterilization, 10 mg/L of ampicillin (Sigma-Aldrich) is added. In order to make the solid medium, the mixture is supplemented with 15 g/L of agar (Becton, Dickinson and Company) before the sterilization thereof. The plates were incubated at 37°C for 16 hours. Then, a colony of each clone was inoculated in 5 mL of LB/ampicillin and the cultures were incubated at 37°C and 220 rpm for 16 hours. The plasmid of each culture was then extracted using the commercial *Miniprep* kit (NucleoSpin Plasmid, Macherey Nagel). This DNA was used to transform competent S. *cerevisiae* BJ5465 cells (*Yeast transformation kit,* Sigma-Aldrich) according to protocol, which were plated in solid SC medium supplemented with 15 g/L of agar, and incubated at 30°C for 3 days. Individual colonies were transferred to 5 mL of SC in 50 mL tubes. These cultures were used to carry out fermentation in 100 mL flasks, as described in the protocol of the first *re-screening.*

### 3. Thermostability measurement

The kinetic thermostability of the different UPO variants, e.g. PaDa-I, was evaluated based on its T₅₀, which is defined as the temperature at which the enzyme loses 50% of activity after 10 mins of incubation. In triplicate, 50 µL of enzymatic supernatant diluted to 1:10 was added to thermal cycler plates (*Multiply PCR plate,* Sarstedt, Germany). The plates were sealed and were incubated 10 mins in a thermo cycler (*Thermocycler Mycycler,* BioRad) programmed with the following temperature gradients (in °C): 30.0; 31.4; 34.8; 39.3; 45.3; 49.9; 55; 56.8; 59.9; 64.3; 70.3; 75; 78.1; and 80.
The plates were then incubated on ice for 10 mins and another 5 mins at room temperature.

Lastly, 20 µL of incubated sample was transferred to new plates measuring the activity, as described in section 1.1.

### Example 1: obtaining the unspecific peroxygenase of the present invention

5 evolution generations were made by *natural drift* with the PaDa-I variant as a parental (obtained as described in Molina-Espeja P. et al., 2014 and which corresponds to the sequence SEQ ID NO: 7 which encodes the amino acid SEQ ID NO: 8).

### 1.1 First generation

A total of 3 mutagenic libraries of -500 clones were built varying the *Taq* polymerase (Sigma-Aldrich)/MnCl₂ ratio, each one from the pJRpsn*upo*1 PaDa-I plasmid obtained from the pJRoC30 plasmid and the nucleic acid encoding PaDa-I with the sequence SEQ ID NO: 7, as indicated in Molina-Espeja P. et al., 2014.

The library L1 corresponded to a low mutation rate (1 to 3 mutations/kb) and was generated from the plasmid pJRpsn*upo*1 PaDa-I generated as indicated in Molina-Espeja P. et al., 2014, carrying out a mutagenic PCR in the following conditions:

**Table 2. Conditions of the mutagenic PCR of library 1 (L1) of the first UPO1 generation.**

| | |
|---|---|
| **Oligonucleotide RMLN** | 0.09 µM |
| **Oligonucleotide RMLC** | 0.09 µM |
| **cDNA (pJRpsn*upo*1 clone PaDa-I)** | 7 ng |
| **dNTPs** | 0.3 mM |
| **MgCl₂** | 1.5 mM |
| **MnCl₂** | 0.01 mM |
| **DMSO** | 3% |
| ***Taq* polymerase buffer (10X)** | 5 µL |
| ***Taq* polymerase** | 2.5 U |
| **Complete with sterile milli-Q water up to** | 50 µL |

The PCR program used was: 95°C for 2 mins (1 cycle); 94°C for 45 s, 53°C for 45 s, 74°C for 3 mins (28 cycles) and 74°C for 10 mins (1 cycle).

The oligonucleotide referred to hereinafter a "RMLN" has the sequence SEQ ID NO: 9 (5'-CCTCTATACTTTAACGTCAAGG-3'); and the oligonucleotide referred to hereinafter as "RMLC" has the sequence SEQ ID NO: 10 (5'- GCTTACATTCACGCCCT CCC -3).

The library L2 was generated from the plasmid pJRpsn*upo*1 clone PaDa-I, with a higher concentration of oligonucleotides and dNTPs, carrying out mutagenic PCR in the conditions that are summarized in the following table:

**Table 3. Conditions of the mutagenic PCR of library 2 (L2) of the first UPO1 generation.**

| | |
|---|---|
| **Oligonucleotide RMLN** | 0.25 µM |
| **Oligonucleotide RMLC** | 0.25 µM |
| **cDNA (pJRpsn*upo*1 clone PaDa-I)** | 5 ng |
| **dNTPs** | 1 mM |
| **MgCl₂** | 1.5 mM |
| **MnCl₂** | 0.01 mM |
| **DMSO** | 3% |
| ***Taq* polymerase buffer (10X)** | 5 µL |
| ***Taq* polymerase** | 2.5 U |
| **Complete with sterile milli-Q water up to** | 50 µL |

The PCR program used was: 95°C for 2 mins (1 cycle); 94°C for 45 s, 53°C for 45 s, 74°C for 3 mins (28 cycles) and 74°C for 10 mins (1 cycle).

The library L3 was generated from the plasmid pJRpsn*upo*1 clone PaDa-I, with a greater concentration of oligonucleotides and dNTPs, carrying out the mutagenic PCR in the conditions that are summarized in the following table:

**Table 4. Conditions of the mutagenic PCR of library 3 (L3) of the first UPO1 generation.**

| | |
|---|---|
| **Oligonucleotide RMLN** | 0.09 µM |
| **Oligonucleotide RMLC** | 0.09 µM |
| **cDNA (pJRpsn*upo*1 clone PaDa-I)** | 5 ng |
| **dNTPs** | 0.2 mM |
| **dTTP** | 0.8 mM |
| **dCTP** | 0.8 mM |
| **MgCl₂** | 1.5 mM |
| **MnCl₂** | 0.25 mM |
| **DMSO** | 3% |
| ***Taq* polymerase buffer (10X)** | 5 µL |
| ***Taq* polymerase** | 2.5 U |
| **Complete with sterile milli-Q water up to** | 50 µL |

The PCR program used was: 95°C for 2 mins (1 cycle); 94°C for 45 s, 53°C for 45 s, 74°C for 3 mins (28 cycles) and 74°C for 10 mins (1 cycle).

The library L3 presented unbalanced amounts of dNTP (0.2 mM dATP and dGTP and 1 mM dTTP and dCTP) along with a greater concentration of MnCl₂, which increased the mutagenic load, as well as the percentage of transitions compared to transversions (Wong *et* al., 2006).

400 ng of each of the PCR reactions were mixed with 200 ng of the pJRoC30 plasmid previously linearized with the restriction enzymes with BamHly and Xhol (New England Biolabs) and transformed into competent S. *cerevisiae* cells following the kit manufacturer's instructions (commercial kit *Yeast Transformation Kit,* Sigma-Aldrich). The transformed cells were inoculated in SC plates supplemented with 15 g/L of agar at 30°C for 3 days.

The *S*. *cerevisiae* BJ5465 strain (ATCC 208289, LGC Promochem) was generally used to express native and evolved genes, artificially cloned in the pJRoC30 plasmid. This strain was auxotrophic for uracil, leucine, tryptophan and histidine, also being deficient in protease (CH1), which makes it suitable for heterologous protein expression and secretion. The complete genotype thereof is: MATa *ura3-52 trp1 leu2-delta1 his3-delta200 pep4::HIS3 prb1-delta1.6R can1*GAL.ln all the cases in which *S*. *cerevisiae* has been used in this work, it has always been the aforementioned strain, unless stated otherwise.

For each one of the clones selected, the ABTS activity was measured as explained in section 2.1 of the analysis methods. The mutagenic landscapes showed that 37.5, 25 and 30.5 % of the clones of the libraries L1, L2 and L3, respectively, were inactive clones, i.e. with an activity lower than 10% of the parental type.

A total of 47 clones of the library L1 that showed neutral activity relative to the parental (between 1.16 and 0.87); 73 clones of the library L2 (between 1.13 and 0.87) and 43 clones of the library L3 (between 1.13 and 0.88) were selected for a second mutagenic cycle. The cut-off values were established in approximate margins of 1.2-0.9 of the parental activity, in order to select mutants that had combined properties in relation to stability and latent activity/ies (cf. Kaltenbach M. et al., 2014).

### 1.2 Second generation

A second generation was prepared using as parental the 163 clones selected from the first generation. A mutagenic *StEP* (*Staggered Extension Process*) was carried out on these new clones using the product of the plasmid DNA extraction using the *NucleoSpin Plasmid* (Macherey-Nagel) jointly obtained from the clones selected from the three mutagenic libraries of the first cycle, as *template* of the PCR.

Briefly, a new library of 500 clones was built carrying out a mutagenic *StEP with Taq* polymerase lacking MnCl₂, where a mixture containing the plasmids of the clones selected in the first generation was used as DNA template (**Table 5**). To extract the plasmids, 20 µL of cell culture of the 163 selected clones were transferred to *deep-well* plates with 96 wells that contained 150 µL of SC. They were incubated at 30°C, 300 rpm and 80% relative humidity (Minitron, Infors) for 24 hrs. Once this time had elapsed, 200 µL of YPD medium was added to each one of the clones, incubating them for 24 hrs under the same conditions. The YPD medium contains 20 g/L of peptone, 10 g/L of yeast extract, 2% of D-glucose (Sigma-Aldrich), 0.2% of L-adenine hemisulfate; at pH 6.5 and 25 mg/mL of chloramphenicol. The cultures of all the clones were mixed and transferred into centrifuge tubes (Beckman coulter), being centrifuged at 3000 g for 10 mins to collect the cells. Once this time had elapsed, the supernatant was discarded and the cells were again resuspended with 150 µL of Solution 1 (kit *Zymoprep Yeast Plasmid Miniprep,* Zymo Research) and 2 µL of zymolyase per each mL of culture, incubating it for 90 minutes at 37°C. Then, 150 µL of Solution 2 and Solution 3 (kit *Zymoprep Yeast Plasmid Miniprep*, Zymo Research) per culture was successively added, centrifuging the tubes at 10,000 g for 10 mins, discarding the pellet, following the instructions provided in the kit. The supernatant was transferred into new tubes in which 400 µL of isopropyl alcohol was added per each mL of culture, centrifuging the tubes again at 10,000 g for 20 mins.

The isopropyl alcohol was removed and the *zymoprep* product was resuspended in 35 µL of TE buffer (1 mM ethylenediaminetetraacetic acid, EDTA, 10 mM Tris-HCl at pH 8) (kit *Zymoprep Yeast Plasmid Miniprep*, Zymo Research) per each mL of culture. 10 µL of *Zymoprep* product were transformed in 100 µL of ultracompetent *E.coli* XL2-Blue cells (Stratagene) following the thermal shock protocol provided by the manufacturer. The transformation product was transferred to 50 mL tubes containing 10 mL of LB/ampicillin, incubating them at 37°C and 220 rpm for 16 hours (*Minitron-INFORS.* Biogen, Spain). Once this time had elapsed, the plasmids were extracted by *miniprep* using the kit *NucleoSpin Plasmid* (Macherey-Nagel), following the instructions provided by the manufacturer.

The complete genotype of the *E.coli* XL2-Blue strain used is: *endA1 supE44 thi-1 hsdR17 recA1 gyrA96 relA1 lac* [F' *proAB laclqZΔM15* Tn*10* (Tetr) Amy Camr]. Whenever *E*. *coli* was used in this work, the strain was this one, unless stated otherwise.

**Table 5. Conditions of the mutagenic StEP amplification reaction of the second generation.**

| | |
|---|---|
| **Oligonucleotide RMLN** | 0.09 µM |
| **Oligonucleotide RMLC** | 0.09 µM |
| **cDNA (pJRpsn*upo*1 clone PaDa-I)** | 6.16 ng |
| **dNTPs** | 0.3 mM |
| **MgCl₂** | 1.5 mM |
| **DMSO** | 3% |
| ***Taq* polymerase buffer (10X)** | 5 µL |
| ***Taq* polymerase** | 2.5 U |
| **Complete with sterile milli-Q water up to** | 50 µL |

The PCR program used was: 95°C for 5 mins (1 cycle); 94°C for 30 s, 55°C for 20 s (90 cycles).

The transformation of *S.cerevisiae* cells with the library, expression of the enzyme and measurement of the activity were carried out as described in the first generation. The mutagenic landscapes showed 25.3% of inactive clones.

Unlike the first generation where the clones with activity comprised between 1.16 and 0.87 of the parental activity were selected, 147 clones with a relative activity against ABTS between 1.05 and 0.92 (zone ND1) and a total of 174 clones with a relative activity between 1.3 and 2.0 (zone ND2) were selected. This second set of clones was selected with the aim that, by having a greater but not excessively increased activity, they would have a greater number of mutations, favoring an increase in the variability of the different clones.

### 1.3 Third generation

The clones of the two chosen regions in the previous section were independently subjected to a new mutagenesis cycle, carrying out a subsequent joint extraction of the variants as described in the second generation.

Briefly, two new independent libraries of 500 clones were - generated, each one based on the 2 regions selected in the previous cycle. The plasmids were extracted following the protocol described in the previous generation. The mutagenic PCR was carried out with the polymerase *Mutazyme II*, setting a mean mutation rate that introduces 4.5 to 9 mutations/kb (**Table 6**).

**Table 6. Conditions of the mutagenic PCR amplification reaction of the third generation.**

| | |
|---|---|
| **Oligonucleotide RMLN** | 0.37 µM |
| **Oligonucleotide RMLC** | 0.37 µM |
| **cDNA (pJRpsn*upo*1 clone PaDa-I)** | 2,737 ng |
| **dNTPs** | 0.8 mM |
| **DMSO** | 3% |
| ***Mutazyme II* polymerase buffer (10X)** | 5 µL |
| ***Mutazyme II* polymerase** | 2.5 U |
| **Complete with sterile milli-Q water up to** | 50 µL |

The PCR program used was: 95°C for 2 mins (1 cycle); 94°C for 45 s, 53°C for 45 s, 74°C for 3 mins (28 cycles) and 74°C for 10 mins (1 cycle).

The transformation of *S.cerevisiae* cells with the library, expression of the enzyme and measurement of the activity were carried out as described in the first generation. The analysis of the activities of the mutagenic libraries showed a percentage of inactive clones of 30.1% for the library from ND1 and 34.4% for ND2.

A total of 96 clones with a relative activity to the parental between 1.09 and 0.91 were selected for a fourth generation.

### 1.4 Fourth generation

The clones selected in the previous generation were mixed, carrying out a subsequent joint extraction of the variants as described in the second generation. The product was subjected to a new evolution cycle with *Taq* polymerase (Sigma-Aldrich), introducing a low mutation rate (1 to 3 mutations/kb).

Briefly, the plasmids from the clones selected in the third generation were extracted following the same protocol that was used in the second generation. The PCR reaction mix, as well as the temperature conditions, was based on library 1 of the first generation (**Table 2**).

The transformation of *S.cerevisiae* cells with the library, expression of the enzyme and measurement of the activity were carried out as described in the first generation. The analysis of the activities measured showed 51% of inactive clones.

A total of 65 neutral clones with a relative activity to the parental between 1.09 and 0.87 were selected for the next generation.

### 1.5 Fifth generation

The clones selected in the fourth generation were mixed, carrying out a subsequent joint extraction of the variants as described in the second generation.

The PCR reaction mix, as well as the temperature conditions, was based on library 1 of the first generation (**Table 2**).

The transformation of *S.cerevisiae* cells with the library, expression of the enzyme and measurement of the activity were carried out as described in the first generation. The mutagenic landscape showed that 37.8% of clones were inactive.

A total of 33 clones from the last generation with an activity between 1.02 and 0.98 against ABTS (measured according to section 2) were selected to evaluate the activity thereof against different substrates to characterize them in a first and second re-*screening*, successively (section 2.2 and 2.3 of the analysis method)

Thus, the peroxidase activity of the enzyme with ABTS was evaluated (according to section 1.1 of the analysis method), and the peroxygenase activity with NBD (according to section 1.2 of the analysis method), in addition to evaluating the activity of the clones for styrene epoxidation (according to section 1.3 of the analysis method). All these were evaluated with spectrophotometric measurements at different wavelengths, as previously described, except for the activity measurement against styrene.

The results of the different activities of the different clones enabled "clone 7" to be selected, a clone which had peroxygenase activities against NBD and styrene greater than all the clones selected after the fifth generation of UPO1 mutants.

### 1.6. Characterization of clone 7

The identified "Clone 7" was sequenced using an automatic sequencer with an ABI 3730 DNA Analyzer capillary electrophoresis system (Applied Biosystems) of the Secugen S.L. Sequencing Service (CIB, Madrid). The samples were prepared using 15 µL of 100 ng/µL plasmid, 1.5 µL of 5 µM oligonucleotides and the primers of sequence SEQ ID NO: 9, SEQ ID NO: 10, SEQ ID NO: 14 and SEQ ID NO: 15. Thus, it was concluded that "clone 7" has the sequence SEQ ID NO: 11.

In addition, a structural analysis of the mutations found in the sequenced "Clone 7" was carried out, in which the mutations Met127Ile and Phe191 Leu were identified. The 3D structure was generated with the *Open-Source* molecular visualization system PymolTM 1.6.x based on the crystallographic structure of the enzyme UPO1 (PDB code: 2Y0R). It was found that the methionine at position 127 established a hydrogen bond with one of the carboxyl groups of the heme group, a bond that is preserved when the residue changes to isoleucine. Furthermore, the phenylalanine at position 191, changed for a leucine, regulates, along with the phenylalanine 76, the entrance of substrates in the heme channel.

The results obtained showed that there was a high variability with regards to the activity linked to oxygen transfer in the clone originally designated as "Clone 7".

"Clone 7" showed a substantially improved activity against styrene and NBD as a substrate with respect to other clones analyzed.

When the ratios between the peroxidase activity measured by ABTS (according to section 1.1 of the analysis method) and the peroxygenase activity (oxygen transfer) measured by NBD (according to section 1.2 of the analysis method) and styrene (according to section 1.3 of the analysis methods) were analyzed, a substantial change of the enzymatic activity towards peroxygenase-type reactions that reached practically a 50% increase was observed:

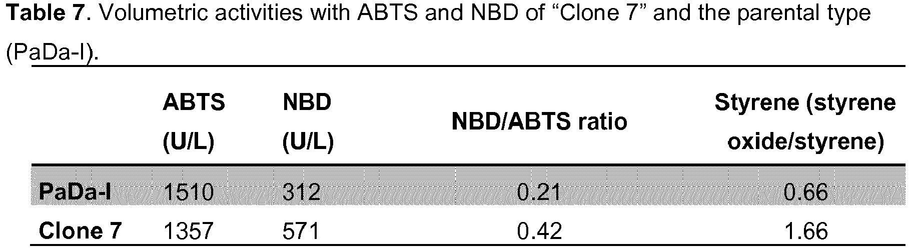

PaDa-I, which presents point mutations that are different to those introduced in "Clone 7" was generated according to Molina-Espeja et al., 2014.

These data show that this substantial increase in the oxygen transfer activity is related to the increase of activity against NBD, of approximately 83%.

Upon verifying its activity on styrene epoxidation, surprising results were obtained, with an increase of 250% in the case of the enzyme of the invention compared to PaDa-I, which lacks the two point mutations at positions 127 and 191.

Therefore, the inclusion of the mutations 127 and 191 (located at a turn-type motif and at an α-helix-type motif, respectively) leads to the remarkable promotion of its peroxygenase activity and to substantially reduce its peroxidase activity, which is unsuitable for specific hydroxylation reactions.

### Example 2: Enzyme immobilized in a solid support

### 2.1. Introduction of mutation S221C in the sequence of the enzyme

The mutation S264C was introduced in the sequence SEQ ID NO: 7 of PaDa-I as well as the sequence SEQ ID NO: 11 of "Clone 7". This residue was selected from among all the Ser residues that were found on the surface of the enzyme because it is the furthest from the active site would not interfere with any of the 5 previous PaDa-I mutations (V57A-L67F-V75I-I248V-F311L) and was also not found close to any other Cys residue with which it could react, forming disulfide bonds that affect the secondary structure of the enzyme. Upon complying with all these prerequisites, it was easier to guarantee that the mutation aimed to immobilize the enzyme did not interfere with the catalytic activities thereof.

The steps for immobilizing PaDa-I are described below.

The PaDa-I variant with sequence SEQ ID NO: 7 was used as a template to introduce the mutation S264C in the sequence SEQ ID NO: 8 through directed mutagenesis. Two high-fidelity PCR reactions were carried out in a final volume of 50 µL which contained: (i) 3% of DMSO, 0.5 µM of RMLN (SEQ ID NO: 9= 5'-CCTCTATACTTTAACGTCAAGG-3'), 0.5 µM of CYS-REV (SEQ ID NO: 12, 5'-CATACGGCTGAATTGGAAAAAACACCGT GCAGCATCCATATCTAG-3'), 1 mM dNTPs (each one 0.25 mM), 0.02 U/µL of iProof DNA of the polymerase, and 0.2 ng/µL of template or (ii) 3% of DMSO, 0.5 µM of CYS-DIR (5'-3') (SEQ ID NO: 13= 5'-CTAGATATGGATGCTGCACGGTGTTTTTTCCAATTCAGCCGTATG-3'), 0.5 µM of RMLC (SEQ ID NO: 10= 5'- GCTTACATTCACGCCCT CCC -3'), 1 mM dNTPs (each one 0.25 mM), 0.02 U/µL of iProof DNA polimerase, and 0.2 ng/µL of template. The following PCR parameters were used for each reaction: (i) 98°C for 30 s (1 cycle), 98°C for 10 s, 47°C for 25 s, 72°C for 35 s (28 cycles), and 72°C for 10 mins (1 cycle); (ii) 98°C for 30 s (1 cycle), 98°C for 10 s, 52°C for 25 s, 72°C for 20 s (28 cycles), and 72°C for 10 minutes (1 cycle). The mutated products of the PCR were mixed with the linearized vector pJRoC30 (in a ratio of PCR product /linearized plasmid of 2:1) and it was transformed into competent *S*. *cerevisiae* BJ5465 cells. Due to the length of the primers used, overlapping areas were created that flanked each fragment, of approximately 50 base pairs (pb), which maximized the efficiency of the DNA processing between the fragments by the yeast through IVOE (Alcalde, 2010).

### 2.2. DNA sequencing

The plasmid that contained the gene PaDa-I S264C was sequenced using an automated sequencer ABI 3730 DNA Analyzer/Applied Biosystems of Secugen S.L. (CIB, Madrid).

### 2.3. Single-point directed covalent immobilization

Before immobilization, the modified enzyme was incubated for 1 hr in a potassium phosphate buffer at pH 7.0 containing 25 mM DTT. The excess DTT was eliminated by gel chromatography using prepacked PD-10 column (GE Healthcare). Immobilization was carried out by incubating the modified enzyme (8 mL) with a Thiol-Sepharose® 4B (1 g) matrix at room temperature for 72 hrs. After immobilization, the biocatalyst was thoroughly washed with buffer in order to eliminate any non-specific binding.

Steps 2.1 to 2.3 were repeated, with the same primers, in order to obtain "Clone 7" modified by the introduction of the mutation S221C and immobilization in the Thiol-Sepharose® 4B matrix.

### Example 3: characterization of the immobilized mutant UPO

### Characterization of the soluble clones PaDa-I Cys and Clone 7 Cys

The activity of the clones PaDa-I, PaDa-I S264C (PaDa-I Cys), Clone 7 and Clone 7 S264C (Clone 7 Cys) was measured as described in sections 1.1 and 1.2 of analysis methods, obtaining the results presented in the following table.

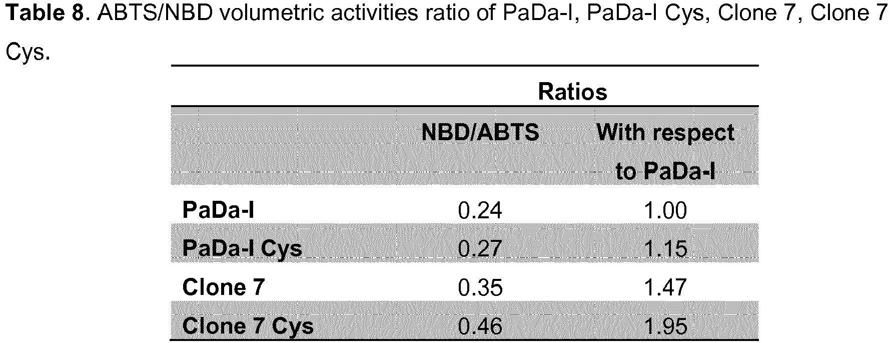

The measurement of ABTS activity was carried out at end time following the protocol: 50 mg of immobilized enzyme in a matrix were weighed on an *eppendorf* tube with a filter. 500 µL of the reaction mixture described in section 1.1 of analysis methods was added on said filter. The measurement was carried out following the method previously described in section 1.1 with the difference that the incubation time was 10 mins instead of 5 mins, given the higher amount of enzyme used, and that it was filtered before the absorbance was measured.

The measurement of the NBD activity was also carried out following the protocol described in section 1.2.

Additionally, the kinetic ABTS activity was determined following the protocol below:
50 mg of the immobilized enzyme in a matrix were weighed and between 1 and 2 mL of ABTS reaction mixture was added to these. 100-200 µL were collected on a regular basis and the absorbance was measured at 418 nm. The absorbance linear increase zone was selected and the slope thereof was found. The activity was determined based on the calculated slope.

The results provided an immobilization efficiency of 20% with an activity of 0.5 U/g for Clone 7 Cys, indicating that the immobilization does not affect the enzymatic activity.

### Bibliography

### Non-patent literature

Alcalde, M., "Mutagenesis protocols in Saccharomyces cerevisiae by in vivo overlap extension", 2010, Methods in Molecular Biology, vol. 634, pages 3-14;
Altschul et al., "Basic local alignment search tool.", 1990, J. Mol. Biol, vol. 215, pages 403-410;
Higgins et al., "CLUSTAL V: improved software for multiple sequence alignment, 1992,
CABIOS, 8(2), pages 189-191;
Hofrichter M. et al.,"Oxidations catalyzed by fungal peroxygenases", Curr. Opin. Chem. Biol, 2014, 19, pages 116-125;
Kaltenbach M. et al., "Generation of effective libraries by neutral drift. In:Directed evolution library creation", Gillam et al editors. Humana press, pp: 69-81;
Mate D.M., et al., "The pocket manual of directed evolution: Tips and tricks. In: Biotechnology of Microbial Enzymes: Production, Biocatalysis and Industrial Applications", 2016, Brahmachari, Demain and Adrio Eds. Elsevier, pages 185-214.
Molina-Espeja P. et al., "Directed Evolution of Unspecific Peroxygenase from Agrocybe aegerita", Applied and Environmental Microbiology, 2014, 80(11), 3496-3507;
Ullrich R. et al., "Novel haloperoxidase from the agaric basidiomycete Agrocybe aegerita oxidizes aryl alcohols and aldehydes", Appl Environ Microbiol., 2004, 70(8), pages 4575-4581;y
Wong, T. S. et al., "The diversity challenge in directed protein evolution", Comb. Chem. High T. Scr., 2004, vol. 9, pages 271-288.

## Claims

1. A modified unspecific peroxygenase enzyme (UPO) EC 1.11.2.1 comprising:
(a) a sequence having at least an 80% of sequence identity with the sequence SEQ ID NO: 2; or
(b) a fragment of a sequence as defined in (a), said fragment maintaining the peroxygenase enzymatic activity;
and further comprising at least one mutation in the turn-type motif defined by the amino acids 104 to 130 of the sequence SEQ ID NO: 2 and at least one mutation in the α-helix-type motif defined by the amino acids 186 to 196 of the sequence SEQ ID NO: 2.

2. The unspecific peroxygenase enzyme according to claim 1, comprising sequence SEQ ID NO: 2, said sequence comprising at least one mutation in the turn-type motif defined by the amino acids 104 to 130 and at least one mutation in the α-helix-type motif defined by the amino acids 186 to 196 of the sequence SEQ ID NO:2.

3. The unspecific peroxygenase enzyme according to any of claims 1-2, comprising sequence SEQ ID NO: 2, a mutation in position 127 and a mutation in position 191, the list of positions referring to SEQ ID NO: 2.

4. The unspecific peroxygenase enzyme according to any of claims 1-3, wherein the mutation is a conservative amino acid substitution.

5. The unspecific peroxygenase enzyme according to any of claims 1-4, wherein the mutation in each of the motifs consists of the substitution by a non-polar amino acid.

6. The modified unspecific peroxygenase enzyme according to any of claims 1-5, further comprising the substitution of at least one Ser residue by one Cys residue.

7. The modified unspecific peroxygenase enzyme according to any of claims 1-6, further comprising the Ser221 Cys mutation.

8. The unspecific peroxygenase enzyme according to any of claims 1-7, which is selected from the group consisting of:
(c) a sequence having at least an 80% of sequence identity with the sequence SEQ ID NO: 3;
(d) a fragment of the sequence (c) provided that the fragment maintains the enzymatic activity and mutations in the positions Met127Ile and Phe191 Leu of SEQ ID NO: 3;
(e) a sequence having at least an 80% of sequence identity with the sequence SEQ ID NO: 4; and
(f) a fragment of the sequence (e) provided that the fragment maintains the enzymatic activity and the mutations in the positions Met127Ile, Phe191 Leu and Ser221Cys of SEQ ID NO: 4.

9. The modified unspecific peroxygenase enzyme according to claim 8, which has the sequence SEQ ID NO: 3 or SEQ ID NO: 4.

10. The modified unspecific peroxygenase enzyme according to any of claims 1-9, comprising a signal peptide at the N-terminal end.

11. The modified unspecific peroxygenase enzyme according to any of claims 1-10 which is immobilized in a support.

12. A nucleic acid encoding an unspecific peroxygenase enzyme as defined in any of claims 1-10.

13. An expression cassette or expression vector comprising the nucleic acid according to claim 12 operatively linked to an expression promotor.

14. A kit comprising the unspecific peroxygenase enzyme as defined in any of claims 1-11, the nucleic acid as defined in claim 12, the expression cassette and/or the expression vector as defined in claim 13.

15. Use of a modified unspecific peroxygenase enzyme as defined in any of claims 1 to 10 as a catalyst, preferably as a catalyst of a styrene oxidation reaction.


SEQUENCE LISTING

<110>  Repsol SA

<120>  Peroxigenasa inespecífica modificada y usos de la misma

<130>  P3816EP00

<160>  15

<170>  PatentIn version 3.5

<210>  1
<211>  371
<212>  PRT
<213>  Agrocybe aegerita

<400>  1

Met Lys Tyr Phe Pro Leu Phe Pro Thr Leu Val Phe Ala Ala Arg Val
1               5                   10                  15

Val Ala Phe Pro Ala Tyr Ala Ser Leu Ala Gly Leu Ser Gln Gln Glu
            20                  25                  30

Leu Asp Ala Ile Ile Pro Thr Leu Glu Ala Arg Glu Pro Gly Leu Pro
        35                  40                  45

Pro Gly Pro Leu Glu Asn Ser Ser Ala Lys Leu Val Asn Asp Glu Ala
    50                  55                  60

His Pro Trp Lys Pro Leu Arg Pro Gly Asp Ile Arg Gly Pro Cys Pro
65                  70                  75                  80

Gly Leu Asn Thr Leu Ala Ser His Gly Tyr Leu Pro Arg Asn Gly Val
                85                  90                  95

Ala Thr Pro Val Gln Ile Ile Asn Ala Val Gln Glu Gly Leu Asn Phe
            100                 105                 110

Asp Asn Gln Ala Ala Val Phe Ala Thr Tyr Ala Ala His Leu Val Asp
        115                 120                 125

Gly Asn Leu Ile Thr Asp Leu Leu Ser Ile Gly Arg Lys Thr Arg Leu
    130                 135                 140

Thr Gly Pro Asp Pro Pro Pro Pro Ala Ser Val Gly Gly Leu Asn Glu
145                 150                 155                 160

His Gly Thr Phe Glu Gly Asp Ala Ser Met Thr Arg Gly Asp Ala Phe
                165                 170                 175

Phe Gly Asn Asn His Asp Phe Asn Glu Thr Leu Phe Glu Gln Leu Val
            180                 185                 190

Asp Tyr Ser Asn Arg Phe Gly Gly Gly Lys Tyr Asn Leu Thr Val Ala
        195                 200                 205

Gly Glu Leu Arg Phe Lys Arg Ile Gln Asp Ser Ile Ala Thr Asn Pro
    210                 215                 220

Asn Phe Ser Phe Val Asp Phe Arg Phe Phe Thr Ala Tyr Gly Glu Thr
225                 230                 235                 240

Thr Phe Pro Ala Asn Leu Phe Val Asp Gly Arg Arg Asp Asp Gly Gln
                245                 250                 255

Leu Asp Met Asp Ala Ala Arg Ser Phe Phe Gln Phe Ser Arg Met Pro
            260                 265                 270

Asp Asp Phe Phe Arg Ala Pro Ser Pro Arg Ser Gly Thr Gly Val Glu
        275                 280                 285

Val Val Ile Gln Ala His Pro Met Gln Pro Gly Arg Asn Val Gly Lys
    290                 295                 300

Ile Asn Ser Tyr Thr Val Asp Pro Thr Ser Ser Asp Phe Ser Thr Pro
305                 310                 315                 320

Cys Leu Met Tyr Glu Lys Phe Val Asn Ile Thr Val Lys Ser Leu Tyr
                325                 330                 335

Pro Asn Pro Thr Val Gln Leu Arg Lys Ala Leu Asn Thr Asn Leu Asp
            340                 345                 350

Phe Phe Phe Gln Gly Val Ala Ala Gly Cys Thr Gln Val Phe Pro Tyr
        355                 360                 365

Gly Arg Asp
    370

<210>  2
<211>  328
<212>  PRT
<213>  Agrocybe aegerita

<400>  2

Glu Pro Gly Leu Pro Pro Gly Pro Leu Glu Asn Ser Ser Ala Lys Leu
1               5                   10                  15

Val Asn Asp Glu Ala His Pro Trp Lys Pro Leu Arg Pro Gly Asp Ile
            20                  25                  30

Arg Gly Pro Cys Pro Gly Leu Asn Thr Leu Ala Ser His Gly Tyr Leu
        35                  40                  45

Pro Arg Asn Gly Val Ala Thr Pro Val Gln Ile Ile Asn Ala Val Gln
    50                  55                  60

Glu Gly Leu Asn Phe Asp Asn Gln Ala Ala Val Phe Ala Thr Tyr Ala
65                  70                  75                  80

Ala His Leu Val Asp Gly Asn Leu Ile Thr Asp Leu Leu Ser Ile Gly
                85                  90                  95

Arg Lys Thr Arg Leu Thr Gly Pro Asp Pro Pro Pro Pro Ala Ser Val
            100                 105                 110

Gly Gly Leu Asn Glu His Gly Thr Phe Glu Gly Asp Ala Ser Met Thr
        115                 120                 125

Arg Gly Asp Ala Phe Phe Gly Asn Asn His Asp Phe Asn Glu Thr Leu
    130                 135                 140

Phe Glu Gln Leu Val Asp Tyr Ser Asn Arg Phe Gly Gly Gly Lys Tyr
145                 150                 155                 160

Asn Leu Thr Val Ala Gly Glu Leu Arg Phe Lys Arg Ile Gln Asp Ser
                165                 170                 175

Ile Ala Thr Asn Pro Asn Phe Ser Phe Val Asp Phe Arg Phe Phe Thr
            180                 185                 190

Ala Tyr Gly Glu Thr Thr Phe Pro Ala Asn Leu Phe Val Asp Gly Arg
        195                 200                 205

Arg Asp Asp Gly Gln Leu Asp Met Asp Ala Ala Arg Ser Phe Phe Gln
    210                 215                 220

Phe Ser Arg Met Pro Asp Asp Phe Phe Arg Ala Pro Ser Pro Arg Ser
225                 230                 235                 240

Gly Thr Gly Val Glu Val Val Ile Gln Ala His Pro Met Gln Pro Gly
                245                 250                 255

Arg Asn Val Gly Lys Ile Asn Ser Tyr Thr Val Asp Pro Thr Ser Ser
            260                 265                 270

Asp Phe Ser Thr Pro Cys Leu Met Tyr Glu Lys Phe Val Asn Ile Thr
        275                 280                 285

Val Lys Ser Leu Tyr Pro Asn Pro Thr Val Gln Leu Arg Lys Ala Leu
    290                 295                 300

Asn Thr Asn Leu Asp Phe Phe Phe Gln Gly Val Ala Ala Gly Cys Thr
305                 310                 315                 320

Gln Val Phe Pro Tyr Gly Arg Asp
                325

<210> 3
<211> 328
<212> PRT
<213> Agrocybe aegerita

<400> 3

Glu Pro Gly Leu Pro Pro Gly Pro Leu Glu Asn Ser Ser Ala Lys Leu
1               5                   10                  15

Val Asn Asp Glu Ala His Pro Trp Lys Pro Leu Arg Pro Gly Asp Ile
            20                  25                  30

Arg Gly Pro Cys Pro Gly Leu Asn Thr Leu Ala Ser His Gly Tyr Leu
        35                  40                  45

Pro Arg Asn Gly Val Ala Thr Pro Val Gln Ile Ile Asn Ala Val Gln
    50                  55                  60

Glu Gly Leu Asn Phe Asp Asn Gln Ala Ala Val Phe Ala Thr Tyr Ala
65                  70                  75                  80

Ala His Leu Val Asp Gly Asn Leu Ile Thr Asp Leu Leu Ser Ile Gly
                85                  90                  95

Arg Lys Thr Arg Leu Thr Gly Pro Asp Pro Pro Pro Pro Ala Ser Val
            100                 105                 110

Gly Gly Leu Asn Glu His Gly Thr Phe Glu Gly Asp Ala Ser Ile Thr
        115                 120                 125

Arg Gly Asp Ala Phe Phe Gly Asn Asn His Asp Phe Asn Glu Thr Leu
    130                 135                 140

Phe Glu Gln Leu Val Asp Tyr Ser Asn Arg Phe Gly Gly Gly Lys Tyr
145                 150                 155                 160

Asn Leu Thr Val Ala Gly Glu Leu Arg Phe Lys Arg Ile Gln Asp Ser
                165                 170                 175

Ile Ala Thr Asn Pro Asn Phe Ser Phe Val Asp Phe Arg Phe Leu Thr
            180                 185                 190

Ala Tyr Gly Glu Thr Thr Phe Pro Ala Asn Leu Phe Val Asp Gly Arg
        195                 200                 205

Arg Asp Asp Gly Gln Leu Asp Met Asp Ala Ala Arg Ser Phe Phe Gln
    210                 215                 220

Phe Ser Arg Met Pro Asp Asp Phe Phe Arg Ala Pro Ser Pro Arg Ser
225                 230                 235                 240

Gly Thr Gly Val Glu Val Val Ile Gln Ala His Pro Met Gln Pro Gly
                245                 250                 255

Arg Asn Val Gly Lys Ile Asn Ser Tyr Thr Val Asp Pro Thr Ser Ser
            260                 265                 270

Asp Phe Ser Thr Pro Cys Leu Met Tyr Glu Lys Phe Val Asn Ile Thr
        275                 280                 285

Val Lys Ser Leu Tyr Pro Asn Pro Thr Val Gln Leu Arg Lys Ala Leu
    290                 295                 300

Asn Thr Asn Leu Asp Phe Phe Phe Gln Gly Val Ala Ala Gly Cys Thr
305                 310                 315                 320

Gln Val Phe Pro Tyr Gly Arg Asp
                325

<210>  4
<211>  328
<212>  PRT
<213>  Agrocybe aegerita

<400>  4

Glu Pro Gly Leu Pro Pro Gly Pro Leu Glu Asn Ser Ser Ala Lys Leu
1               5                   10                  15

Val Asn Asp Glu Ala His Pro Trp Lys Pro Leu Arg Pro Gly Asp Ile
            20                  25                  30

Arg Gly Pro Cys Pro Gly Leu Asn Thr Leu Ala Ser His Gly Tyr Leu
        35                  40                  45

Pro Arg Asn Gly Val Ala Thr Pro Val Gln Ile Ile Asn Ala Val Gln
    50                  55                  60

Glu Gly Leu Asn Phe Asp Asn Gln Ala Ala Val Phe Ala Thr Tyr Ala
65                  70                  75                  80

Ala His Leu Val Asp Gly Asn Leu Ile Thr Asp Leu Leu Ser Ile Gly
                85                  90                  95

Arg Lys Thr Arg Leu Thr Gly Pro Asp Pro Pro Pro Pro Ala Ser Val
            100                 105                 110

Gly Gly Leu Asn Glu His Gly Thr Phe Glu Gly Asp Ala Ser Ile Thr
        115                 120                 125

Arg Gly Asp Ala Phe Phe Gly Asn Asn His Asp Phe Asn Glu Thr Leu
    130                 135                 140

Phe Glu Gln Leu Val Asp Tyr Ser Asn Arg Phe Gly Gly Gly Lys Tyr
145                 150                 155                 160

Asn Leu Thr Val Ala Gly Glu Leu Arg Phe Lys Arg Ile Gln Asp Ser
                165                 170                 175

Ile Ala Thr Asn Pro Asn Phe Ser Phe Val Asp Phe Arg Phe Leu Thr
            180                 185                 190

Ala Tyr Gly Glu Thr Thr Phe Pro Ala Asn Leu Phe Val Asp Gly Arg
        195                 200                 205

Arg Asp Asp Gly Gln Leu Asp Met Asp Ala Ala Arg Cys Phe Phe Gln
    210                 215                 220

Phe Ser Arg Met Pro Asp Asp Phe Phe Arg Ala Pro Ser Pro Arg Ser
225                 230                 235                 240

Gly Thr Gly Val Glu Val Val Ile Gln Ala His Pro Met Gln Pro Gly
                245                 250                 255

Arg Asn Val Gly Lys Ile Asn Ser Tyr Thr Val Asp Pro Thr Ser Ser
            260                 265                 270

Asp Phe Ser Thr Pro Cys Leu Met Tyr Glu Lys Phe Val Asn Ile Thr
        275                 280                 285

Val Lys Ser Leu Tyr Pro Asn Pro Thr Val Gln Leu Arg Lys Ala Leu
    290                 295                 300

Asn Thr Asn Leu Asp Phe Phe Phe Gln Gly Val Ala Ala Gly Cys Thr
305                 310                 315                 320

Gln Val Phe Pro Tyr Gly Arg Asp
                325

<210>  5
<211>  43
<212>  PRT
<213>  Agrocybe aegerita

<400>  5

Met Lys Tyr Phe Pro Leu Phe Pro Thr Leu Val Phe Ala Ala Arg Val
1               5                   10                  15

Val Ala Phe Pro Ala Tyr Ala Ser Leu Ala Gly Leu Ser Gln Gln Glu
            20                  25                  30

Leu Asp Ala Ile Ile Pro Thr Leu Glu Ala Arg
        35                  40

<210>  6
<211>  129
<212>  DNA
<213>  Agrocybe aegerita

<400>  6
atgaaatatt ttcccctgtt cccaaccttg gtcttcgcag cgagggtcgt tgcttttcct      60

gcctacgcct cattggccgg cctcagccag caggaattgg acgctataat cccaacactc     120

gaggcccga                                                             129

<210>  7
<211>  1116
<212>  DNA
<213>  Artificial Sequence

<220>
<223>  PaDa-I

<400>  7
atgaaatatt ttcccctgtt cccaaccttg gtctacgcag tgggggtcgt tgcttttcct      60

gactacgcct cattggccgg cctcagccag caggaattgg acgctataat cccaacactc     120

gaggcccgag agccaggatt acctcctggt cctctcgaga atagctctgc aaagttggtg     180

aacgacgagg ctcacccatg gaagccgctt cgacctggcg atattcgtgg accttgccct     240

ggtctcaata ctctggcatc tcacgggtac ctcccgagaa atggcgttgc aaccccggcg     300

caaataataa acgcggttca ggaaggattc aatttcgaca atcaagccgc aatcttcgcc     360

acatatgcgg cccaccttgt ggacggcaat ctcattacgg acttgctgag catcggacgc 420

aagacgcggc tcactgggcc tgatccacca ccccccgctt ccgttggtgg actcaatgag 480

catggcacct tcgaaggcga cgccagtatg acccgaggtg acgcattctt tggcaacaac 540

cacgatttca atgagacgct cttcgaacag ttggttgact acagcaaccg atttggagga 600

ggaaaataca atcttaccgt cgcgggggag ctccgtttca agcgcattca agactccatt 660

gcgaccaacc ccaatttctc ctttgttgac tttaggttct ttactgctta cggcgagacc 720

accttccccg cgaatctttt tgtggatggg cgcagggacg acggccagct agatatggat 780

gctgcacgga gttttttcca attcagccgt atgcctgacg atttcttccg cgcacccagc 840

ccgagaagtg gcacaggagt cgaggtagtt gtacaggctc atcctatgca gcccggaaga 900

aatgtcggca agatcaacag ctacaccgtc gacccaacat cctctgactt ttccaccccc 960

tgcttgatgt acgagaaatt cgtcaacata acggtcaagt cactctaccc gaatccgacg 1020

gtgcagcttc gcaaagccct taatacgaat ctcgatttct tattccaggg agtcgccgct 1080

ggatgtaccc aggtcttccc atacgggcga gattga 1116

<210> 8
<211> 371
<212> PRT
<213> Artificial Sequence

<220>
<223> PaDa-I

<400> 8

Met Lys Tyr Phe Pro Leu Phe Pro Thr Leu Val Tyr Ala Val Gly Val
1               5                   10                  15

Val Ala Phe Pro Asp Tyr Ala Ser Leu Ala Gly Leu Ser Gln Gln Glu
            20                  25                  30

Leu Asp Ala Ile Ile Pro Thr Leu Glu Ala Arg Glu Pro Gly Leu Pro
        35                  40                  45

Pro Gly Pro Leu Glu Asn Ser Ser Ala Lys Leu Val Asn Asp Glu Ala
    50                  55                  60

His Pro Trp Lys Pro Leu Arg Pro Gly Asp Ile Arg Gly Pro Cys Pro
65                  70                  75                  80

Gly Leu Asn Thr Leu Ala Ser His Gly Tyr Leu Pro Arg Asn Gly Val
                85                  90                  95

Ala Thr Pro Ala Gln Ile Ile Asn Ala Val Gln Glu Gly Phe Asn Phe
            100                 105                 110

Asp Asn Gln Ala Ala Ile Phe Ala Thr Tyr Ala Ala His Leu Val Asp
        115                 120                 125

Gly Asn Leu Ile Thr Asp Leu Leu Ser Ile Gly Arg Lys Thr Arg Leu
    130                 135                 140

Thr Gly Pro Asp Pro Pro Pro Pro Ala Ser Val Gly Gly Leu Asn Glu
145                 150                 155                 160

His Gly Thr Phe Glu Gly Asp Ala Ser Met Thr Arg Gly Asp Ala Phe
                165                 170                 175

Phe Gly Asn Asn His Asp Phe Asn Glu Thr Leu Phe Glu Gln Leu Val
            180                 185                 190

Asp Tyr Ser Asn Arg Phe Gly Gly Gly Lys Tyr Asn Leu Thr Val Ala
        195                 200                 205

Gly Glu Leu Arg Phe Lys Arg Ile Gln Asp Ser Ile Ala Thr Asn Pro
    210                 215                 220

Asn Phe Ser Phe Val Asp Phe Arg Phe Phe Thr Ala Tyr Gly Glu Thr
225                 230                 235                 240

Thr Phe Pro Ala Asn Leu Phe Val Asp Gly Arg Arg Asp Asp Gly Gln
                245                 250                 255

Leu Asp Met Asp Ala Ala Arg Ser Phe Phe Gln Phe Ser Arg Met Pro
            260                 265                 270

Asp Asp Phe Phe Arg Ala Pro Ser Pro Arg Ser Gly Thr Gly Val Glu
        275                 280                 285

Val Val Val Gln Ala His Pro Met Gln Pro Gly Arg Asn Val Gly Lys
    290                 295                 300

Ile Asn Ser Tyr Thr Val Asp Pro Thr Ser Ser Asp Phe Ser Thr Pro
305                 310                 315                 320

Cys Leu Met Tyr Glu Lys Phe Val Asn Ile Thr Val Lys Ser Leu Tyr
                325                 330                 335

Pro Asn Pro Thr Val Gln Leu Arg Lys Ala Leu Asn Thr Asn Leu Asp
            340                 345                 350

Phe Leu Phe Gln Gly Val Ala Ala Gly Cys Thr Gln Val Phe Pro Tyr

355                 360                 365

Gly Arg Asp
    370

<210>  9
<211>  22
<212>  DNA
<213>  Artificial Sequence

<220>
<223>  primer

<400>  9
cctctatact ttaacgtcaa gg                                                22

<210>  10
<211>  20
<212>  DNA
<213>  Artificial Sequence

<220>
<223>  primer

<400>  10
gcttacattc acgccctccc                                                   20

<210>  11
<211>  1122
<212>  DNA
<213>  Artificial Sequence

<220>
<223>  nucleotide sequence of the enzyme in clone 7

<400>  11
atgaaatatt ttcccctgtt cccaaccttg gtctacgcag tgggggtcgt tgcttttcct      60

gactacgcct cattggccgg cctcagccag caggaattgg acgctataat cccaacactc     120

gaggcccgag agccaggatt acctcctggt cctctcgaga atagctctgc aaagttggtg     180

aacgacgagg ctcacccatg gaagccgctt cgacctggcg atattcgtgg accttgccct     240

ggtctcaata ctctggcatc tcacgggtac ctcccgagaa atggcgttgc aaccccggcg     300

caaataataa acgcggttca ggaaggattc aatttcgaca atcaagccgc aatcttcgcc     360

acatatgcgg cccaccttgt ggacggcaat ctcattacgg acttgctgag catcggacgc     420

aagacgcggc tcactgggcc tgatccacca cccccgcctt ccgttggtgg actcaatgag     480

catggcacct tcgaaggcga cgccagtata acccgaggtg acgcattctt tggcaacaac     540

cacgatttca atgagacgct cttcgaacag ttggttgact acagcaaccg atttggagga     600

ggaaaataca atcttaccgt cgcgggggag ctccgtttca agcgcattca agactccatt     660

gcgaccaacc ccaatttctc ctttgttgac tttaggttcc ttactgctta cggcgagacc     720

accttccccg cgaatctttt tgtggatggg cgcagggacg acggccagct agatatggat 780

gctgcacgga gttttttcca attcagccgt atgcctgacg atttcttccg cgcacccagc 840

ccgagaagtg gcacaggagt cgaggtagtt gtacaggctc atcctatgca gcccggaaga 900

aatgtcggca agatcaacag ctacaccgtc gacccaacat cctctgactt ttccaccccc 960

tgcttgatgt acgagaaatt cgtcaacata acggtcaagt cactctaccc gaatccgacg 1020

gtgcagcttc gcaaagccct taatacgaat ctcgatttct tattccaggg agtcgccgct 1080

ggatgtaccc aggtcttccc atacgggcga gattgacgaa gg 1122

<210> 12
<211> 45
<212> DNA
<213> Artificial Sequence

<220>
<223> primer

<400> 12
catacggctg aattggaaaa aacaccgtgc agcatccata tctag 45

<210> 13
<211> 45
<212> DNA
<213> Artificial Sequence

<220>
<223> primer

<400> 13
ctagatatgg atgctgcacg gtgttttttc caattcagcc gtatg 45

<210> 14
<211> 21
<212> DNA
<213> Artificial Sequence

<220>
<223> primer

<400> 14
gaaggcgacg ccagtatgac c 21

<210> 15
<211> 21
<212> DNA
<213> Artificial Sequence

<220>
<223> primer

<400> 15
ggtcatactg gcgtcgcctt c 21